# EUROPEAN PATENT APPLICATION

(11) **EP 1 609 460 A1**
(43) Date of publication of application: **28.12.2005**
(21) Application number: 04253768.8
(22) Date of filing: 24.06.2004
(51) Int. Cl.: A61K 7/48

(54) **Skin care compositions comprising fatty material having a melting temperature range of 21-40 C**

(71) Applicant: THE PROCTER & GAMBLE COMPANY, Cincinnati, Ohio 45202 (US)
(72) Inventor: Crook, Teresa Barbara, Camberley Surrey GU17 9LY (GB); Vanoosthuyze, Kristina Emma Inge, Working Surrey GU21 4XD (GB)
(74) Representative: L'Huillier, Florent Charles

(57) **Abstract**

Skin care compositions comprising fatty material having a specific melting temperature range are disclosed. The fatty materials used are solid at room temperature, but melts readily on contact with skin. The fatty materials are combined with high level of humectant to provide highly moisturizing composition having improved application feel for the user.

## Description

### FIELD

The present invention relates to leave-on skin care moisturizing compositions.

### BACKGROUND

Numerous skin moisturizing formulations have been proposed in the past with diverse success. Whereas the primary function of a moisturizing skin care composition is to introduce water and/or help skin reduce water loss, other desired properties have proven harder to achieve. In particular, there is a need for highly moisturizing skin care compositions having enjoyable application properties.

The following documents are cited as background art:
- US5,972,858 - US5,891,451 - US5,855,893 - US5,084,563 - US5,731,450;
- WO2004/039338 - WO2004/004674 - WO2003/101407 - WO2003/084497 - W02003/039508 - WO2001/82889 - WO2001/85104 - WO98/25585 - WO96/10388;
- EP421,333 - EP345,082;
- DE32 46 265A1 - DE298 10 730 U1.

### SUMMARY

The present invention is directed to an oil-in-water moisturizing composition comprising:
a) from 4.0% to 15% by weight of the composition of fatty material having a melting point range comprised between 21°C and 40°C; and
b) at least 8% by weight of the total composition of skin moisturizing polyhydric alcohol.

The compositions of the invention comprise less than 10 % by weight of soap and are thus less irritating to the skin than soap-based cleansing composition.

The viscosity of the compositions ranges from about 50,000 to about 150,000 cps (measured at 25°C on a Brookfield Viscometer RVT at 5 rpm with Helipath using TC spindle, 1 mn). This viscosity was found to provide an attractively thick, but not too thick, cream which can be easily applied on the skin by consumers.

The compositions of the invention combine high hydration benefit (due to the synergy between high glycerine level and occlusivity of the fatty material) with an application profile that is distinctly different from regular body lotion with the same amount of glycerine. The slow melting of the fatty material (e.g. cocoa butter) during rub in on the skin leads to a long application which gives a luxurious and pampering sensation. After dry down, the skin has a very soft and velvety afterfeel and is non sticky/tacky, despite the high levels of glycerine, niacinamide (if present) and total oil level.

Preferably, the composition is packaged in a jar having a large neck that allows the consumer to directly scoop the composition out of the container using his fingers.

The compositions also have the benefit of looking very rich and luxurious, and have the same rich and luxurious feel when scooped out of the jar.

These and other features, aspects, and advantages of the present invention will become evident to those skilled in the art from a reading of the present disclosure.

### DETAILED DESCRIPTION

While the specification concludes with claims which particularly point out and distinctly claim the invention, it is believed the present invention will be better understood from the following description.

All percentages are by weight of total composition unless specifically stated otherwise. All ratios are weight ratios unless specifically stated otherwise.

### A. The fatty materials

The composition of the present invention includes from 4.0% to 15% by weight of the composition of fatty material (which can be a single material or a mixture of materials) having a melting point range comprised between about 21°C to about 40°C. Preferably the compositions comprise from 4.0% to 10% of fatty material according to the invention.

The melting point of fatty materials is usually defined as a range of temperature. The melting point range of fatty materials is normally available in the literature, in particular from supplier's literature. If not, it can be measured by conventional methods, for example by the European Pharmacopoeia Method 2.2.15.

The fatty materials of the invention are solid at room temperature, but melts readily on contact with skin. The preferred melting point range of the fatty materials of the invention is comprised between about 23°C and about 38°C, more preferably between about 25°C and about 36°C.

The preferred fatty materials for use in the present invention are designated in the art as vegetable butter. The preferred vegetable butters of the invention are cocoa butter, shea butter, mango kernel butter and mixtures thereof. Cocoa butter has a reported melting point range of from 31°C to 35°C. Shea butter has a reported melting point of 31°C. Mango kernel butter has a reported melting point range of from 28°C to 36°C.

The applicant has found that at sufficiently high level (>4%), the fatty materials claimed provide the compositions with a long and luxurious application profile, as well as a rich sensation when scooping the product out of the jar. Once molten on the skin, the fatty material forms an occluding layer on the skin synergistically preventing evaporation of the water that is bound by the humectant (e.g. glycerine).

Vegetable butters such as cocoa butter are complex polymorphic fats. This means that they can solidify into a number of crystal arrangements. Those tiny fat crystals can reflect light, giving it a glossy appearance. This also contributes to the luxurious look of the composition.

Compositions with level of fatty material above 15% are normally too solid to be easily applied and do not provide the required viscosity. The viscosity of the compositions ranges from about 50,000 to about 150,000 cps, preferably from about 80,000 to about 120,000 cps (measured at 25°C on a Brookfield Viscometer RVT at 5 rpm with Helipath using TC spindle, 1mn). As is common practice, 1 mn means that three successive measurements separated by one rotation each are made within 1 minute of the start of the measurement and averaged to give a viscosity value.

### B. Polyhydric alcohol humectant

The compositions of the invention comprise at least 10% by weight of polyhydric alcohol humectant (which can be a single material or a mixture). Suitable polyhydric alcohol humectants for use herein include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexane triol (e.g., 1,2,6-hexanetriol), trimethylol propane, neopentyl glycol, glycerine, ethoxylated glycerine and propoxylated glycerine.

Preferred polyhydric alcohol humectants of the present invention are polyhydric alcohols with 3 to 9 carbon atoms in the molecule. Suitable polyhydric alcohols include glycerine, butylene glycol, propylene glycol, dipropylene glycol, polyethylene glycol and derivatives thereof, hexane triol, ethoxylated glycerine and propoxylated glycerine, or mixtures thereof. More preferred for use in the present invention is glycerine.

The compositions of the present invention comprise 8% or more, preferably from 9% to 25%, more preferably from 10% to 20%, still more preferably from 10.5 to 17.5% of polyhydric alcohol humectant. The preferred polyhydric alcohol is glycerine and the compositions preferably comprise at least 5%, preferably at least 10% or more of glycerine by weight of the total composition as polyhydric alcohol humectant.

### C. Oil-in-water emulsion

The cosmetic compositions herein are in the form of oil-in-water (O/W) emulsion. O/W emulsions were found to provide the best results in terms of moisturization of skin and application feel. The total level of oil phase components in the compositions of the invention is typically from about 5% to about 25%, preferably from about 7.5% to about 20% by weight of the total composition.

The water phase may comprise water only or a combination of water and one or more water soluble or dispersible ingredients. The total level of water phase components in the compositions of the invention is typically from about 75% to about 95%, preferably from about 60% to about 92.5% by weight of the total composition.

The oil phase preferably comprises oily materials such as natural or synthetic oils selected from mineral, vegetable, and animal oils, fats and waxes, fatty acid esters, fatty alcohols, fatty acids and mixtures thereof. Preferred for use herein are for example, saturated and unsaturated fatty alcohols such as behenyl alcohol, cetyl alcohol and stearyl alcohol and hydrocarbons such as mineral oils or petrolatum.

The oil phase may further comprise oil-soluble skin care actives. Non-limiting examples of oil-soluble skin care actives suitable for use herein include ceramides, cholesterols, fatty acids, vitamin E or its derivatives, oil-soluble vitamin B₃ compounds, or mixtures thereof. Another oil-soluble skin care active suitable for use herein comprise the oil-soluble vitamin B₃ compounds including "non-vasodilating" esters of nicotinic acid, examples of which include tocopherol nicotinate. As used herein, the term "non-vasodilating" means that the ester does not commonly yield a visible flushing response after application to the skin in the composition of interest.

The present compositions may further comprise a silicone phase. The silicone phase can comprise one or more silicone components such as silicone fluids, gums, and mixtures thereof. If present, the, or each, silicone phase generally comprises from 0.1% to 20%, preferably from 0.2% to 10%, more preferably from 0.3% to 5%, of the composition.

The present compositions herein may comprise an emulsifier and/or surfactant, generally to help disperse and suspend the discontinuous phase within the continuous phase. Known or conventional surfactants can be used in the composition, provided that the selected agent is chemically and physically compatible with essential components of the composition, and provides the desired characteristics. The compositions of the present invention preferably comprise from 0.05% to 15% of a surfactant or mixture of surfactants. The exact surfactant or surfactant mixture chosen will depend upon the pH of the composition and the other components present. Surfactants suitable for use herein include non-ionic, cationic, anionic, zwitterionic, amphoteric surfactants, or mixtures thereof, preferably non-ionic surfactants.

The compositions comprise less than 10% by weight of soap and are thus less irritating to the skin than cleansing composition. By "soap" we mean alkali and alkaline earth metal salts of long chain fatty acids such as a lauric-stearic acid mixture. Soaps are harsh on the skin and are preferably present in less than 5% by weight, even more preferably less than 1% by weight of the composition. Some soap may be present as part of the emulsifying system, usually at levels of from 0.1% to 0.5%. The compositions may comprise no soap at all.

### D. Soft Olive Wax

The compositions of the present invention may advantageously further include Soft Olive Wax, which is a mixture of hydrogenated olive oil and olive oil. Soft Olive Wax further enhances the application feel of the composition and has conditioning properties. It absorbs on the skin very well, unlike the fatty material claimed which are more occlusive and tends to remain on the surface of the skin. This provides a composition with a good balance of occlusive and penetration properties.

### E. Cetyl acetate and acetylated lanolin alcohol

The compositions of the present invention may advantageously further include a mixture of cetyl acetate and acetylated lanolin alcohol. This mixture makes the composition easier to spread on the skin, and helps delivering the enjoyable application feel of the invention. This is especially useful with the fatty materials claimed which are normally very heavy and difficult to spread on the skin. The mixture also has emollient properties. Commercially, a preferred mixture of cetyl acetate and acetylated lanolin alcohol is supplied by Croda under the TradeName Crodalan LA. Typical but non-limiting levels are from about 0.2% to about 2% by weight of the composition.

### F. Emollients

The topical compositions of the present invention may also comprise additional skin emollients other than the fatty materials claimed and Soft Olive Wax. A further emollient suitable for use in the composition of the present invention is petrolatum. Also suitable are branched chain hydrocarbons having a weight average molecular weight of from 100 to 15,000. Suitable branched chain hydrocarbons for use herein include isododecane, isohexadecane, isoeicosane, isooctahexacontane, isohexapentacontahectane, isopentacontaoctactane, and mixture thereof.

When present, these additional emollients may be in the compositions at levels of from about 0.1% to about 10% by weight of the composition.

### G. Skin care actives

The cosmetic compositions of the present invention may additionally comprise a skin care active. Skin care actives suitable for use herein comprise panthenol and its derivatives, retinoids and their derivatives, salicylic acid or its derivatives, humectants, amino acids and their derivatives, vitamin C and its derivatives or mixtures thereof. Skin care actives are useful for providing visual improvements in skin appearance or condition following multiple topical applications of the composition to the skin. This "chronic" benefit is particularly desirable as it evens the texture of skin and so increases skin sheen by providing a desquamatory, keratolytic and rejuvenating effect and/or moisturization.

The skin care active may comprise panthenol or its derivatives. The panthenol and its derivatives include D-panthenol ([R]-2,4-dihydroxy-N-[3-hydroxypropyl)]-3,3-dimethylbutamide), DL-panthenol, calcium pantothenate, royal jelly, panthetine, pantotheine, panthenyl ethyl ether, pangamic acid, pyridoxin, pantoyl lactose and Vitamin B complex. More preferably, the composition herein comprises panthenol. The panthenol or its derivative is preferably used in an amount of from about 0.1% to about 5%, more preferably from about 0.2% to about 3%.

A further class of skin care actives useful herein comprises retinoids. As used herein, "retinoid" includes all natural and/or synthetic analogs of Vitamin A or retinal-like compounds which possess the biological activity of Vitamin A in the skin as well as the geometric isomers and stereoisomers of these compounds. Preferred retinoids comprise retinol, retinyl palmitate, retinyl acetate, retinyl proprionate, retinal or mixtures thereof. More preferred are retinol, retinyl palmitate, or mixtures thereof. The compositions of this invention may contain a safe and effective amount of the retinoid, e.g. from about 0.005% to about 2%, more preferably 0.01% to about 2%, retinoid.

Other possible skin care actives according to the present invention include additional humectants. Suitable humectants useful herein include sodium 2-pyrrolidone-5-carboxylate (NaPCA), amino acids and derivatives, guanidine; glycolic acid and glycolate salts (e.g. ammonium and quaternary alkyl ammonium); lactic acid and lactate salts (e.g. ammonium and quaternary alkyl ammonium); other alpha hydroxy acids such as malic acid, aloe vera in any of its variety of forms (e.g., aloe vera gel); hyaluronic acid, precursors and derivatives thereof (e.g., glucosamine and salt derivatives such as sodium hyaluronate); lactamide monoethanolamine; acetamide monoethanolamine; urea; and mixtures thereof.

Another suitable skin care active is a vitamin C compound. Vitamin C compounds include water-soluble ascorbic acid salts and esters thereof. Vitamin C compounds are particularly useful as skin lightening agents. Suitable examples include magnesium ascorbyl phosphate and the sodium salt of the monophosphate ester of ascorbic acid, commercially available from Roche Vitamins Europe Ltd as Stay-C 50™.

### H. Other optional ingredients

The topical compositions of the present invention may comprise a wide variety of optional components, provided that such optional components are physically and chemically compatible with the essential components described herein, and do not unduly impair stability, efficacy or other use benefits associated with the compositions of the present invention. Optional components may be dispersed, dissolved or the like in the carrier of the present compositions.

Preferably the pH of the cosmetic composition is adjusted to be mild to the skin. Preferably, the pH of the composition of the present invention is not less than about 5.0, more preferably from about 5.0 to about 7.0, more preferably still from about 5.5 to about 6.5.

The compositions of the present invention can also comprise a thickening agent, preferably from about 0.1% to about 5%, more preferably from about 0.1% to about 3%, and most preferably from about 0.25% to about 2%, by weight of the total composition. Non-limiting examples of thickening agents suitable for use herein include cross-linked acrylate copolymers, hydroxyalkylacrylate copolymers, polyacrylamide polymers, natural gum thickeners, or mixtures thereof.

A preferred thickener is a cross-linked co-polymer comprising from 35% to 95%, preferably from 50% to 70%, water-soluble anionic acrylic monomers, from 5% to 65% water-soluble non-ionic acrylate monomers, and from 0.005% to 0.5% bifunctional monomeric crosslinking agent by weight of the cross-linked co-polymer. Commercial examples of co-polymer compositions suitable for use herein include Luvigel™ EM from BASF Corp. and Salcare SC91 ™ from CIBA Speciality Chemicals, Macclesfield, UK.

The compositions of the present invention may optionally include particulate materials. Particulate materials suitable herein include materials that are insoluble in both water and oil with a median particle size of from 1 µm to 50 µm. Suitable particulate materials are organic or organosilicone or inorganic. Representative commercially available examples of useful particulate materials herein are Microthene FN510™, Tospearl 145™, Orgasol 2002™, Nylonpoly WL10™, Dry Flo™ or mixtures thereof. The compositions of the present invention may comprise from about 0.1% to about 5% by weight of particulate materials.

A further optional component may comprise sunscreening agents (e.g. ethylhexyl salicylate (Octisalate), butyl methoxydibenzoylmethane (Avobenzone), phenylbenzimidazole sulfonic acid (Ensulizole), 2-ethylhexyl-2-cyano-3,3-diphenylacrylate (Octocrylene), Zinc Oxide). Generally, the sunscreens can comprise from about 0.5% to about 20% of the compositions useful herein. Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, August 25, 1978.

Further optional ingredients include avocado oil, seaweed/marine extracts, lavender oil extract and jasmine oil.

### I. Presentations - Packaging

The compositions of the present invention are preferably presented to the consumer as a leave-on body lotion composition packaged in jar. By "jar", we mean a container with a neck sufficiently large for the user to scoop the composition out of the jar with his fingers.

The cosmetic compositions of the present invention may also be made into a wide variety of product forms such as are known in the art.

### J. Method of making

The oil-in-water emulsions of the invention can be made following any conventional processes, e.g. a process using two vessels. The method described in the examples sections may be used and adapted to make the compositions of the invention.

### Examples

The following examples further describe and demonstrate the preferred embodiments within the scope of the present invention. The examples are given solely for the purpose of illustration, and are not to be construed as limitations of the present invention since many variations thereof are possible without departing from its spirit and scope.

| **INGREDIENT FUNCTION** | **FORMULA INGREDIENTS** | **1** | **2** | **3** | **4** | **5** |
|---|---|---|---|---|---|---|
| **CARRIER** | DEIONISED WATER | QS | QS | QS | QS | QS |
| **SKIN ACTIVES** | GLYCERINE | 10.0 | 10.0 | 8.5 | 15.0 | 10.0 |
| | NIACINAMIDE | 3.5 | 3.5 | 2.0 | 2.5 | 3.5 |
| | PANTHENOL | 0.5 | 0.5 | 0.5 | 0.5 | 0.5 |
| | TOCOPHEROL ACETATE | 0.25 | 0.25 | 0.25 | 0.25 | 0.25 |
| **VEGETABLE BUTTER** | COCOA BUTTER | 4.0 | 4.0 | 2.0 | -- | -- |
| | SHEA BUTTER | -- | -- | 3.0 | -- | -- |
| | MANGO KERNEL BUTTER | -- | -- | -- | 4.0 | 6.0 |
| **EMOLLIENTS** | SOFT OLIVE WAX | 1.0 | 1.0 | 0.5 | 2.0 | 1.0 |
| | ISOHEXADECANE | 3.0 | 3.0 | 3.5 | 1.5 | 3.0 |
| | ISOPROPYL ISOSTEARATE | 1.50 | 1.50 | 1.50 | 1.50 | 2.0 |
| | COCONUT OIL FRACTIONATED | 0.3 | 0.3 | 0.1 | 0.5 | 0.3 |
| | PETROLATUM | 2.0 | 2.0 | 3.0 | 1.0 | 2.0 |
| | CRODALAN® LA | - | 2.0 | 2.0 | 2.0 | 2.0 |
| **THICKENER / EMULSIFIER** | LUVIGEL® EM | 2.0 | 2.50 | 2.50 | 2.50 | 2.0 |
| | STEARYL ALCOHOL | 0.61 | 0.61 | 0.61 | 0.61 | 0.61 |
| | CETYL ALCOHOL | 0.49 | 0.49 | 0.49 | 0.49 | 0.49 |
| | BEHENYL ALCOHOL | 0.40 | 0.40 | 0.40 | 0.40 | 0.40 |
| | PEG-100 STEARATE | 0.1 | 0.1 | 0.1 | 0.1 | 0.1 |
| | EMULGADE | 0.2 | 0.2 | 0.2 | 0.2 | 0.2 |
| | STEARIC ACID | 0.1 | 0.1 | 0.1 | 0.1 | 0.1 |
| **PARTICLES** | MICROTHENE | 0.5 | 0.5 | 0.5 | 0.5 | 0.5 |
| | DRY FLO PLUS | 1.1 | 1.1 | 1.1 | 1.1 | 1.1 |
| **MISC** | ETHYL PARABEN | 0.15 | 0.15 | 0.15 | 0.15 | 0.15 |
| | PROPYL PARABEN | 0.07 | 0.07 | 0.07 | 0.07 | 0.07 |
| | DISODIUM EDTA | 0.1 | 0.1 | 0.1 | 0.1 | 0.1 |
| | BENZYL ALCOHOL | 0.25 | 0.25 | 0.25 | 0.25 | 0.25 |
| **Viscosity (cps)** | DC™ 1503 | 2.0 | 1.0 | 2.0 | 1.0 | 2.0 |
| | SODIUM HYDROXIDE | 0.011 | 0.011 | 0.011 | 0.011 | 0.011 |
| | PRODEW 400 | 0.1 | 0.1 | 0.1 | 0.1 | 0.1 |
| | PERFUME | 0.4 | 0.6 | 0.6 | 0.6 | 0.6 |
| | | 80200 | 93000 | N.a. | N.a. | N.a |
| LUVIGEL® EM is a Caprylic / Capric Triglyceride and Sodium Acrylate Copolymer, supplied by BASF. | | | | | | |
| CRODALAN® LA is Cetyl Acetate and Acetylated Lanolin alcohol, supplied by Croda Inc. | | | | | | |
| EMULGADE® is a mixture of Cetearyl Glucoside and Cetearyl Alcohol supplied by Cognis. | | | | | | |
| DC® 1503 is a mixture of Dimethicone & Dimethiconol. | | | | | | |
| Prodew® 400 is a mixture of Sodium PCA, Betain, Sorbitol, Alanine, Proline, Serine, Threonine, Arginine, Lysine and Glutamic Acid. | | | | | | |
| The perfume used was a standard perfume. Any perfumes may be used. | | | | | | |

### FORMULATION PROCESS

1. Place oil phase ingredients in a vessel and heat to 75°C
2. Place water phase ingredients in a separate vessel and heat to 75°C
3. Add oil phase ingredients to the water phase vessel and mix well.
4. Homogenise the above mixture to form an emulsion.
5. Cool the above emulsion to 60°C and add the polymeric thickener (Luvigel™ EM) and associated anionic surfactants ( Oleth-3 and Laureth-7 ).
6. Cool emulsion to 55°C and add Sodium Hydroxide solution to neutralise to pH 6-7.
7. Cool mixture to 50°C and add Panthenol, Crodalan™ LA, DC™ 1503, Benzyl Alcohol.
8. Cool mixture to 45°C, add particles and shear to ensure particle dispersion, de- agglomeration and homogeneity.
9. Cool emulsion to 40°C and add perfume.
10. Cool mixture to 35°C while mixing.
11. Product can be then prepared for packaging and labeling.

It is understood that the examples and embodiments described herein are for illustrative purposes only and that various modifications or changes in light thereof will be suggested to one skilled in the art without departing from the scope of the present invention.

## Claims

1. A thick, leave-on skin care moisturizing composition comprising, by weight of the composition:
a) from 4.0% to 15% of fatty material having a melting point range of from 21°C to 40°C; and
b) at least 10% of skin moisturizing polyhydric alcohol;
wherein said composition is an oil-in-water emulsion;
wherein said composition comprises less than 10% of soap; and
wherein the viscosity of the composition ranges from to 50,000 to 150,000 cps (measured at 25°C with a Brookfield Viscometer RVT at 5 rpm with Helipath using TC spindle, 1mn).

2. A composition according to claim 1 wherein said fatty material is a vegetable butter, preferably selected from cocoa butter, shea butter, mango kernel butter and mixtures thereof.

3. A composition according to any of the preceding claims wherein said fatty material is present at level of from 4.0% to 10% by weight.

4. A composition according to any of the preceding claims further comprising Soft Olive Wax.

5. A composition according to any of the preceding claims further comprising a mixture of cetyl acetate and acetylated lanolin alcohol.

6. A composition according to any of the preceding claims wherein the skin moisturizing polyhydric alcohol material is selected from glycerine, butylene glycol, propylene glycol, dipropylene glycol, polyethylene glycol and derivatives thereof, hexane triol, ethoxylated glycerine and propoxylated glycerine, and mixtures thereof.

7. A composition according to any of the preceding claims wherein the composition comprises at least 5% glycerine, preferably at least 10% glycerine, by weight of the composition.

8. A composition according to any of the preceding claims wherein the composition is packaged in a jar having a neck large enough for the user to scoop the composition directly out of the jar with his fingers.

9. A cosmetic method of treating skin wherein a composition according to any of preceding claims is applied to the skin.

10. A cosmetic method according to claim 9 wherein the composition is left on the skin after application.
